# EUROPEAN PATENT APPLICATION

(11) **EP 1 410 836 A2**
(43) Date of publication of application: **21.04.2004**
(21) Application number: 03255806.6
(22) Date of filing: 17.09.2003
(51) Int. Cl.: B01D 53/50, B01D 53/56, B01D 53/60, B01D 53/62, C01B 21/40, C07C 47/22, C01C 3/02, C07C 63/16

(54) **Method for abatement of waste oxide gas emissions**

(30) Priority: 18.10.2002 US 419555 P
(71) Applicant: ROHM AND HAAS COMPANY, Philadelphia, Pennsylvania 19106-2399 (US)
(72) Inventor: Day, James Clarence, 700 Lower State Rd. North Wales, PA19454 (US); Decourcy, Michael Stanley, Houston, Texas 77062 (US); Lonzetta, Charles Michael, Houston, Texas 77059 (US); Myers, Ronald Eugene, Mt. Holly, New Jersey 08060 (US)
(74) Representative: Kent, Venetia Katherine

(57) **Abstract**

The methods of the present invention relate to reducing and eliminating waste oxide gas emissions, produced by a first industrial process, by utilizing the emissions in a second industrial process that either is benefited by or tolerates the components of the waste oxide gas stream. These methods are applicable to numerous combinations of first industrial processes and second industrial processes.

## Description

The present invention is directed to methods for reducing or eliminating waste oxide gases in industrial waste streams. More particularly, the present invention relates to a process for reducing or eliminating nitrogen-based oxides, carbon-based oxides and sulfur-based oxides from the emissions produced by industrial processes. Even more specifically, the methods of the present invention provide methods wherein a process stream, created by a first industrial process, that contains waste oxide gases is introduced into a second industrial process capable of reducing or eliminating the amount of waste oxide gas.

Waste oxide gases ("WOG") are those gases containing nitrogen-based oxides ("NOₓ"), sulfur-based oxides ("SOₓ") or carbon-based oxides ("COₓ"), and may, in fact, contain mixtures of two or more of these. Such waste oxide gases are produced by a variety of processes including chemical processes and combustion processes. When introduced to the environment, waste oxide gases produce undesirable effects. For example, nitrogen-based oxides play a major role in the formation of ozone and are believed to be responsible for the nitric acid component of acid rain. Sulfur-based oxides are associated with acidification of lakes and streams, accelerated corrosion of buildings and monuments, reduced visibility and adverse health effects. Carbon-based oxides, most notably carbon monoxide, may present serious health concerns to the public and have been linked to global warming. Due to these serious adverse environmental and health effects, introduction of waste oxide gases to the environment is strictly regulated by both national and regional agencies. Such regulations are expected to become even more restrictive in the coming years.

Various unsatisfactory methods to reduce the emissions of WOG produced by industrial processes have been introduced previously. However, none of those methods are capable of substantially eliminating waste oxide emissions, and all involve substantial capital, operating and/or maintenance costs. Moreover, such prior art methods may result in reduced process efficiency and they often generate by-products that are as detrimental to the environment as the waste oxide gases themselves. In addition, the prior art methods tend to be conflicting, such that abatement of carbon oxides tends to favor formation of nitrogen oxides and vice versa, resulting in little or no cumulative abatement.

Traditional methods to reduce the emissions of WOG can be broken down into two basic categories: those that inhibit the formation of the WOG ("formation inhibiting methods") and those that destroy the WOG once they are formed ("oxide destroying methods"). In practice, WOG emissions are often controlled using combinations of these two methods to effect the desired reduction in emissions. For example, NOₓ formation may be reduced by the formation-inhibiting method of avoiding high combustion temperatures; NOₓ that is nonetheless formed may then be treated with an oxide-destroying method such as selective catalytic reduction. The abatement methods currently available in the art vary widely in their effectiveness.

EPA Report Number EPA-453/R-93-007 dated January 1993 and entitled "Alternative Control Techniques Document - NOₓ Emissions from Stationary Gas Turbines," describes wet and dry formation-inhibiting methods to reduce waste oxide emissions from turbines. The wet methods involve injecting steam or water into the combustion chamber to reduce the combustion temperature because the rate of formation of NOₓ increases rapidly at combustion temperatures above 1540°C. Such wet methods are not desirable methods of reducing WOG emissions because they reduce combustion efficiency and necessitate the use of more fuel to evince the desired level of combustion. Moreover, such wet techniques are unable to substantially reduce WOG emissions. The dry formation inhibiting methods discussed in this report include lean premixed combustion designs and rich/quench/lean staged combustion designs. Lean premixed combustion involves premixing the fuel and air prior to combustion to create a uniform mixture and reduce or eliminate fuel-rich pockets that often create elevated combustion temperatures and, thus, higher NOₓ emissions. Rich/quench/lean staged combustion involves partially combusting in a fuel rich environment, rapidly quenching the partially combusted mixture and then completing combustion in a low-temperature, fuel-lean environment. This technique reduces NOₓ formation in the fuel-rich stage due to lack of available oxygen and reduces NOₓ formation in the fuel-lean stage due to lower combustion temperatures. Another formation inhibiting method is flue gas recirculation ("FGR"), wherein a portion of the flue gas is returned to the combustion zone to reduce flame temperature and dilute the combustion air supply with the relatively inert flue gas. Negative environmental impacts associated with the various formation inhibiting methods include increased carbon monoxide production and unburned hydrocarbon emissions. In sum, none of the currently available combustion inhibiting methods are capable of substantially reducing or eliminating WOG emissions.

Oxide destroying methods remove WOG from process streams regardless of how the WOG are produced. Oxide destroying methods include selective non-catalytic reduction ("SNCR") and selective catalytic reduction ("SCR"). Unlike combustion controls, SNCR and SCR do not reduce WOG emissions by inhibiting WOG formation, but rather by destroying the waste oxides once formed. Such methods use reducing agents to transform a portion of the WOG to environmentally inert components, such as nitrogen and water. In the SCR abatement method, a waste oxide gas stream is contacted with ammonia and then the ammonia/WOG stream combination is passed through a catalyst bed. Where NOₓ is the waste oxide being abated, the ammonia and NOₓ react to form harmless nitrogen and water.

In the SNCR abatement method, a nitrogen-containing chemical is injected into a WOG stream having a gas temperature in the range of 1600°F to 2100°F. In this temperature range, the injected nitrogen-containing chemical reacts selectively with the WOG in the process stream to create benign by-products without using a catalyst. Where NOₓ is the WOG being processed, for example, SNCR transforms the NOₓ into nitrogen and water. While several nitrogen-containing chemicals have been investigated for use in SNCR systems, the chemicals of primary interest for full-scale industrial applications are ammonia and urea.

While they are able to achieve some level of reduction in WOG emissions, conventional oxide destroying methods, such as those described above, are not without undesirable environmental drawbacks. For instance, use of SNCR leads to release of ammonia and nitrous oxide into the environment. Released ammonia can lead to adverse impacts downstream of the SNCR system, including air heater fouling, plume formation, and contamination of otherwise marketable fly ash. SCR can also have undesirable effects, such as the release of ammonia, enhanced production of undesirable sulfur oxides and high gas-side pressure drops. Moreover, ammonia handling and storage, necessitated by SNCR and SCR methods, presents serious safety concerns; and the systems may also affect plant operations and result in energy penalties by reducing boiler efficiency and consuming power in their operation. Furthermore, the SCR abatement method employs zeolite or precious metal catalysts. Such catalysts are not only expensive to purchase but expensive to dispose of when their useful life has ended. The catalysts are also sensitive to contaminants, such as sulfur-containing compounds, and the system can readily foul (e.g., in-situ formation of ammonium sulfate). Maintenance costs are also increased with oxide destroying methods. Specialized continuous emissions monitoring equipment is often required to monitor the performance of the system.

Various U.S. patents describe other methods of WOG abatement such as recycle-based methods. U.S. Patent No. 5,077,434 to Sarumaru *et al*. (the "'434 patent"), for example, discloses a process comprising subjecting acrylic acid absorber off-gas to a catalytic combustion oxidation process and then recycling the treated off-gas back to the acrylic acid process for use as a diluent. Unfortunately, this recycle method does not reduce or eliminate WOG emissions ; rather, the WOG accumulate to the extent that they are no longer useful as a diluent and at that point must be vented.

Similarly, U.S. Patent No. 4,031,135 to Engelbach *et al*. (the "'135 patent") discloses an acrylic acid production process wherein an absorber off-gas process stream containing waste oxides is directly recycled back to the acrylic acid reactor for use as a diluent. The '135 patent uses the waste oxide containing stream as a cold diluent in an effort to maintain the reactants in the two-stage acrylic acid process in a non-flammable state without the use of copious quantities of steam, a traditional diluent. Like the method of the '434 patent, the method described by the '135 patent does not reduce waste oxide gas emissions.

Therefore, there remains an unaddressed and long-felt need for anefficient method for eliminating or reducing WOG emissions, especially in light of the increased governmental restrictions on such emissions.

Accordingly, provided herein are methods for substantially reducing or eliminating waste oxide gas emissions.

In one aspect of the present invention, there is provided a method for abating waste oxide gases from a waste oxide gas stream, the method comprising:
(a) providing a first industrial process, the first industrial process producing a waste oxide gas stream, the waste oxide gas stream comprising at least one waste oxide gas selected from the group consisting of nitrogen oxides, sulfur oxides and carbon oxides;
(b) providing a second industrial process, the second industrial process being a different process than the first industrial process, the second industrial process abating the quantity of said waste oxide gas stream, from the first industrial process, when said waste oxide gas stream is fed to said second industrial process as a feed stream; and
(c) feeding at least a portion of said waste oxide gas stream, from the first industrial process, as a feed stream, to the second industrial process.

In some embodiments of the present invention, the second industrial process is benefited by the introduction of the waste oxide stream. In such cases, not only are undesirable WOG destroyed, but the second industrial process also benefits. The benefit may be in the form of increased yield or an extension of the second industrial process' catalyst's life.

In other embodiments of the present invention, the second industrial process may be tolerant of the waste oxide gas stream. In such cases, the waste oxides are often converted into inert products, such as water and nitrogen, that may then be vented to the atmosphere with little concern.

In still other embodiments of the present invention, the second industrial process may transform the waste oxides into another, more easily handled, form of waste. For example, in such cases, the waste oxide gas may be converted into a solid waste salt, capable of traditional disposal.

Other and further objects, features and advantages of the methods of the present invention will be apparent from the following description of some embodiments of the invention when viewed in conjunction with the drawings and examples.

A more complete understanding of the present embodiments and advantages thereof may be acquired by referring to the following description taken in conjunction with accompanying Figures 1A, 1B, 2A, 2B, and 3 - 5, in which like reference numbers indicate like features.
Figure 1A is an example of an acrolein production process in which the waste oxide stream is abated with a conventional oxide destroying process.
Figure 1B is an example of an acrolein production process in which the waste oxide stream is abated according to one embodiment of the method of the present invention.
Figure 2A is an example of a hydrogen cyanide production process in which the waste oxide stream is abated according to one embodiment of the method of the present invention.
Figure 2B is an example of a hydrogen cyanide production process in which the waste oxide stream is abated according to another embodiment of the method of the present invention.
Figure 3 is an example of a phthalic anhydride production process in which the waste oxide stream is abated according to one embodiment of the method of the present invention.
Figure 4 is an example of a nitric acid production process in which the waste oxide stream is abated according to one embodiment of the method of the present invention.
Figure 5 is an example of a furnace-based process for producing sulfuric acid in which the waste oxide stream is abated according to one embodiment of the method of the present invention.

The methods of the present invention generally involve taking a WOG stream produced by a first industrial process and introducing that WOG stream into a second industrial process wherein all or a portion of the WOG is abated.

The first industrial process may be any process that produces a waste stream containing waste oxides. Suitable examples of waste gas streams of a first industrial process include: waste gas streams from chemical manufacturing process absorbers such as those used in the production of nitric acid, adipic acid, acrylonitrile, hydrogen cyanide and (meth) acrylic acid; waste gas streams from combustion processes such as engines, boilers, turbines, heaters, incinerators, furnaces, thermal oxidizers and catalytic combustion units; stack gas from air compressors and co-generation systems; waste gas streams from wet oxidation processes; waste gas streams from high-temperature processes, such as cement kilns, iron and steel mills, and glass manufacturing plants; and, off-gas streams from traditional waste oxide abatement systems such as conventional ammonia-based selective catalytic reduction units.

The second industrial process follows the first industrial process and may be any industrial process that is capable of abating all or a portion of the WOG emitted from the first industrial process. Although the second industrial process should not be the same process as the first industrial process, a variety of processes are suitable for use as the second industrial process. The second industrial process most beneficially is one in which one or more of the following components are routinely present: hydrogen, carbon oxides, nitrogen oxides, ammonia, hydrocarbons and oxygen. It is within the ability of one skilled in the art with the benefit of this disclosure to determine a suitable second industrial process to be used to abate the waste oxide gas stream produced by a first industrial process. Nevertheless, suitable examples of the second industrial process include but are not limited to:
a hydrogen cyanide production process, for example, an Andrussow or oxygen-enriched process;
a bleaching process, for example, those wherein NO/NO₂ are used with supplemental air/O₂ to form HNO₃;
a carbon bed desorption process;
an oxidation process, for example, those wherein compounds such as (meth)acrylic acid, phthalic anhydride, (meth)acrylonitrile and (meth)acrolein are produced;
an oxidative dehydrogenation of hydrocarbons process;
an oxygen addition process, for example, a process used to activate phenolic inhibitors in vinyl monomer separation processes to prevent polymer formation;
an ammoxidation process, for example, those used to produce phthalonitrile and (meth)acrylonitrile;
an air stripping process, such as a NOₓ stripping process to produce adipic acid;
a process for the partial oxidation of hydrocarbons, for example, where oxidation is achieved via autothermal reforming of methane;
a sulfuric acid regeneration process;
a reaction of tert-butanol, isobutene, iso-butane, iso-butyraldehyde or the methyl ether of tert-butanol to yield (meth)acrolein and/or (meth)acrylic acid;
a reaction of o-xylene or naphthalene to yield phthalic anhydride;
a reaction of butadiene, for example, to yield maleic anhydride or vinyloxirane; and
a reaction of indanes, for example, to yield products such as anthraquinone.

Choosing an appropriate second industrial process requires consideration of both the volume and composition of the WOG stream produced by the first industrial process.

With respect to composition, if the WOG stream contains components that are detrimental to the second industrial process, those detrimental components will have to be either removed or reduced to levels that will not harm the second industrial process. For instance, where the first industrial process is a coal combustion process, the WOG stream may contain substantial amounts of particulate matter that may not be suitable for the chosen second industrial process. If such particulate matter will be detrimental to the second industrial process, it will have to be removed by some means known in the art such as filtration or electrostatic precipitation prior to the introduction of the WOG stream into the second industrial process. Another example is where the WOG stream contains halogen compounds or metallic compounds that are detrimental to the second industrial processes. For instance, many catalytic processes suitable for use as second industrial processes are sensitive to halogen compounds, in which case such compounds must be removed by some means known in the art prior to the introduction of the WOG stream to the second process. In yet another example, the WOG stream may contain sulfur compounds. Some oxygen-based second industrial processes are sensitive to the presence of sulfur. Therefore, in some embodiments, it is preferred that the concentration of sulfur compounds, including sulfur oxides, be minimized in the WOG stream before it is introduced to the second industrial process. An example of a second industrial process sensitive to such sulfur compounds is a (meth) acrylic acid production process; the life of the oxidation catalyst used in such a process is shortened by exposure to sulfur compounds. Such sulfur compounds may be removed by any means known in the art such as the use of low-sulfur content fuels to inhibit sulfur compound formation or the use of physical removal methods such as electrostatic precipitation, adsorption or scrubbing to remove the sulfur compounds from the WOG stream.

In some embodiments of the present invention, the composition of the WOG stream from the first industrial process will not be detrimental to the second industrial process. For instance, where the second industrial process is a sulfuric acid regeneration process, the presence of sulfur oxides in the WOG stream is easily tolerated and no removal will be necessary. Compounds present in the WOG stream may in fact benefit the second industrial process. By way of example, in the case of the catalytic oxidation of o-xylene or naphthalene to phthalic anhydride, Japanese Patent Abstract JP07039767, "Method For Activating Fluid Catalyst For Phthalic Anhydride Production," teaches that catalyst activity may be improved through exposure to low concentrations of sulfur dioxide.

In some embodiments of the methods of the present invention, it may be advantageous to alter the composition of the WOG stream not only by removing potentially harmful components but by adding desirable components to the WOG stream before the WOG stream is introduced to the second industrial process. For instance, it may be desirable to blend the WOG stream with an oxygen-containing gas, most preferably an oxygen-containing gas comprising greater than about 20% oxygen, before using the WOG stream in an oxygen-based second industrial process.

The volume of the waste oxides in the WOG stream as well as the volume of the WOG stream itself exiting the first industrial process must also be considered. At low waste oxide volumes, the method of the present invention may be used widely and the number of second industrial processes that will produce a substantial reduction in WOG emissions is large. In situations where the volume of waste oxides produced is too large to be abated in a single second industrial process, the WOG stream exiting the first industrial process may be treated in a traditional WOG abatement process, such as SCR or SNCR, before the stream is introduced to the second industrial process. Alternatively, the WOG stream from the first industrial process may be divided and sent to a plurality of second industrial processes for abatement, the number of second industrial processes dependent on the volume of the WOG and the capacities of the second industrial processes.

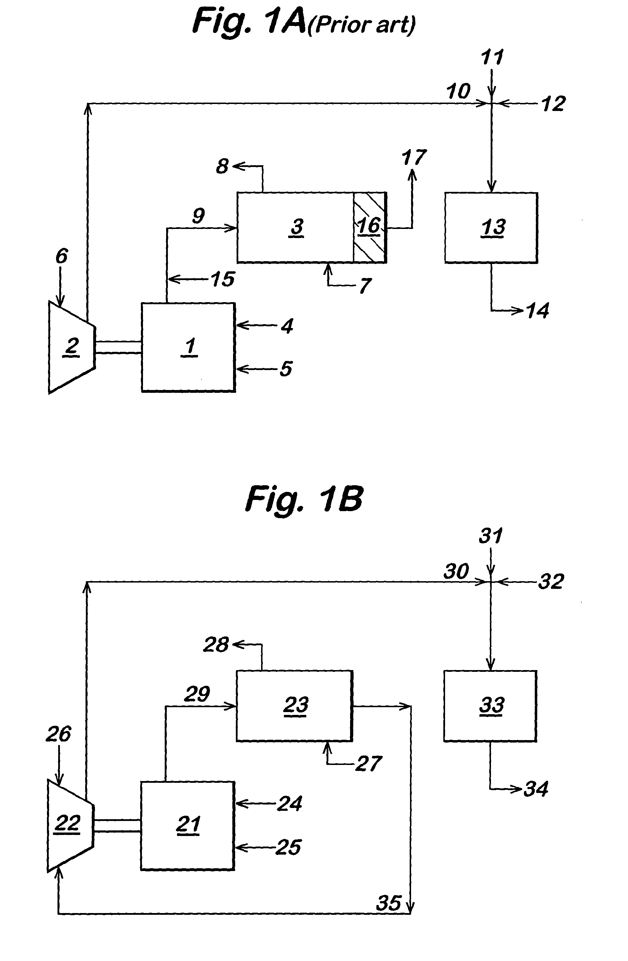

Figures 1B, 2A, 2B, 3, 4 and 5 illustrate various aspects and embodiments of the methods of the present invention. These are nonlimiting examples, and should not be construed to describe all first and second industrial processes to which the methods of the present invention are applicable. With the benefit of this disclosure, one of ordinary skill in the art will recognize additional combinations of first industrial processes and second industrial processes for which the methods of the present invention will be suitable.

Figure 1A illustrates a conventional system for producing acrolein wherein conventional oxidation reactor **13** is fed hydrocarbon stream **12** comprising propylene, diluent stream **11** comprising inerts, and oxygen-containing gas stream **10** to produce product stream **14** comprising acrolein. In the system shown in Figure 1A, diluent stream **11** comprises recycled inert gases from an acrylic acid purification process. The molar feed ratio of propylene:oxygen:inerts fed to oxidation reactor **13** is approximately 1:1.6-2.1:12-16, with the exact feed ratio being dependent on a range of factors including the specific catalyst composition, reactor operating conditions, propylene purity, and the like. The selection of an appropriate feed ratio for a given set of conditions is within the ability of one of ordinary skill in the art.

In addition to oxidation reactor **13**, the system for producing acrolein shown in Figure 1A also includes gas turbine **1**, compressor **2**, heat recovery steam generator **3** and selective catalytic reduction (SCR) unit **16**. Gas turbine **1** combusts methane fuel **4** and atmospheric air **5** to produce shaft work capable of driving compressor **2**. Compressor **2** then intakes atmospheric air at inlet **6**, compresses it, and discharges it via line **10** to be delivered to oxidation reactor **13** as the oxygen-containing gas stream feed.

However, gas turbine **1** produces more than just shaft work, it also acts as a first industrial process, producing hot WOG stream **9** comprising carbon oxides, nitrogen oxides and residual oxygen. WOG stream **9** is discharged to heat recovery steam generator **3**. In heat recovery steam generator **3**, heat from WOG stream **9** is transferred to boiler feed water **7**, thereby cooling WOG stream **9** and producing a process stream comprising steam **8**. Once cooled, WOG stream **9** is processed in selective catalytic reduction unit **16** to reduce the concentration of waste oxides prior to vent. This reduction is accomplished via reaction of the waste oxides with a reducing agent, such as ammonia, added at feed line **15**. After passing through selective catalytic reduction unit **16**, the treated WOG stream is vented to the atmosphere at vent line **17**. Despite the use of selective catalytic reduction unit **16**, a significant quantity of the waste oxides that were initially present in WOG stream **9** when it exited gas turbine **1** are not removed and exit to the atmosphere through vent line **17**.

Figure 1B is comparable to Figure 1A and illustrates a system for producing acrolein similar to that in Figure 1A, however, the WOG stream produced by the gas turbine is treated according to an embodiment of the method of the present invention. In the system shown in Figure 1B, conventional oxidation reactor **33** (the second industrial process according to this embodiment of the method of the present invention) takes hydrocarbon stream **32** comprising propylene, diluent stream **31** comprising inerts, and oxygen-containing gas stream **30** to produce product stream **34** comprising acrolein. In this embodiment, diluent stream **31** comprises recycled inert gases from an acrylic acid purification process. The molar feed ratio of propylene:oxygen:inerts fed to oxidation reactor **33** is generally about 1:1.5-2.5:12-16, with the exact feed ratio being dependent on a range of factors including the specific catalyst composition, reactor operating conditions, propylene purity, and the like. The selection of an appropriate feed ratio for a given set of conditions is within the ability of one of ordinary skill in the art.

In addition to oxidation reactor **33**, the system for producing acrolein shown in Figure 1B also includes gas turbine **21**, compressor **22** and heat recovery steam generator **23**. Gas turbine **21** combusts methane fuel **24** and atmospheric air 25 to produce shaft work capable of driving compressor **22**. However, gas turbine **21** produces more than just shaft work, it also acts as a first industrial process, producing WOG stream **29** comprising carbon oxides, nitrogen oxides, and residual oxygen. WOG stream **29** is discharged to heat recovery steam generator **23**. In heat recovery steam generator **23**, heat from the hot WOG stream is transferred to boiler feed water **27**, thereby cooling WOG stream **29** and producing a process stream comprising steam **28**, which can be vented or used in a process. As WOG stream **29** comprises oxygen in addition to waste oxides, rather than treating it in a traditional SCR system, cooled WOG stream **35** is discharged to the inlet of compressor **22** to be used as an oxygen-containing gas feed to oxidation reactor **33**. Compressor **22** intakes cooled WOG stream **35**, compresses it, and discharges it via line **30** for delivery to oxidation reactor **33** as the oxygen-containing gas stream feed. In alternate embodiments of the present invention, compressor **22** may also be fed additional atmospheric air via process line **26**. In the method of the present invention, oxidation reactor **33** acts as a second industrial process. Cooled WOG stream **35** is sent from compressor **22** to conventional oxidation reactor **33**, and is completely abated, leaving no undesirable waste oxides to be introduced to the environment.

Preferably, the operating conditions of conventional oxidation reactor **33** may be shifted to use this embodiment of the methods of the present invention. For instance, the gas in process line **30**, made up of cooled WOG stream **35**, may generally comprise about 15% by volume oxygen. The reactor feed ratios, expressed as propylene:oxygen:inerts, remain as described above. However, the oxygen content of 15% by volume is lower than that of atmospheric air, generally at least about 20% by volume. Thus, the relative volume of diluent needed will be reduced, and the relative volume of oxygen-containing gas needed will be increased. Again, the selection of an appropriate feed ratio for a given set of conditions is within the ability of one of ordinary skill in the art. It will also be evident to one of ordinary skill that this inventive method applies equally well to the production of alternative oxidation products - such as acrylic acid, methacrolein, or methacrylic acid - and that other hydrocarbons may be substituted for all or a part of the propylene feed in order to produce acrolein or the alternative products. Moreover, pure oxygen may be used in place of atmospheric air where appropriate.

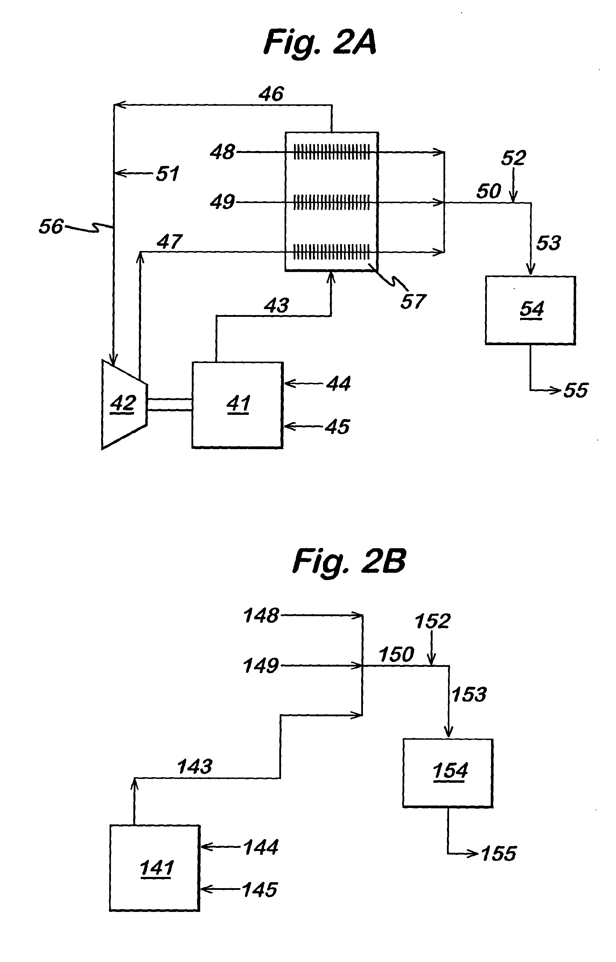

Figure 2A illustrates a system for producing hydrogen cyanide wherein methane, ammonia, and an oxygen-containing gas are ammoxidized over a platinum-based catalyst gauze in a reactor. To supply a sufficient volume of oxygen-containing gas to reactor **54**, a General Electric Model MS5000 gas turbine **41** bums methane **44** and atmospheric air **45** to produce enough shaft work to operate compressor **42**. In addition to producing shaft work, gas turbine **41** also acts as a first industrial process, creating WOG exhaust stream **43**. Despite the use of conventional waste oxide reduction methods such as high secondary air flow and steam injection into the combustion zone of gas turbine **41**, WOG exhaust stream **43** generally comprises **18**-33 ppm NOₓ and 26-116 ppm CO, as well as 17.4 - 17.7% by volume oxygen. This concentration of waste oxides is too great to be vented directly to the atmosphere under the current environmental regulatory scheme.

WOG exhaust stream **43** exits gas turbine **41** at a high temperature, and its heat energy is recovered in heat exchanger **57**. Heat exchanger **57** is used to pre-heat the raw materials to be fed to conventional reactor **54**. Heat exchanger **57** receives ammonia via process line **48**, methane via process line **49** and oxygen-containing gas via compressor outlet **47**. Each of those three raw materials are contained in the tube-side of heat exchanger **57** while WOG exhaust stream **43** is introduced to the shell side of heat exchanger **57**. As a result of the heat exchange, WOG exhaust stream **43** is cooled and exits heat exchanger **57** as cooled WOG exhaust stream **46**. The preheated raw materials are combined into preheated reactor feed **50** when they exit heat exchanger **57**. Mixing devices and filtration systems (not shown) may be beneficially employed to aid in the combination of the raw material streams.

Rather than being sent to a traditional WOG abatement system or vented directly to the environment, in accordance with this embodiment of the methods of the present invention, the oxygen-containing cooled WOG exhaust stream **46** may be routed to compressor suction line **56**. In an alternate embodiment of the present invention, atmospheric air may be added to compressor suction line **56** via process feed line **51**. The oxygen-containing gases, cooled WOG exhaust with or without additional atmospheric air, are fed to compressor **42** via compressor suction line **56**. Compressor **42** vents the compressed oxygen-containing gases through compressor outlet **47** to heat exchanger **57**. Preferably, the components of compressor **42** in contact with waste oxides, such as condensers, rotors, and compressor outlet **47** duct work, are made of corrosion resistant materials such as 300 series stainless steels. Further, in an alternative embodiment, it may be advantageous to cool the compressor suction line, utilize compressor interstage cooling, and/or remove condensate to improve the efficiency of compressor **42**. As described above, the compressed oxygen-containing gases in compressor outlet **47** are preheated in heat exchanger **57** and then combined with preheated methane and ammonia in preheated reactor feed **50**.

Preheated reactor feed **50** is then sent through process line **53** to conventional reactor **54**. In this type of process, the oxygen content of cooled WOG exhaust stream **46** is generally 17 - 18% oxygen versus at least about 20% oxygen normally found in atmospheric air. To compensate for that reduced oxygen concentration, compressor outlet **47** may be preheated to a temperature from 100°C to 200°C higher than the preheat temperature that would have been used in a hydrogen cyanide production system employing only atmospheric air as the oxygen-containing gas in process line **47**. Optionally, the percentage of oxygen in preheated reactor feed **50** may be increased prior to its introduction to reactor **54** by introducing additional oxygen-containing gas to process line **53** via oxygen-containing gas feed line **52**. Where oxygen-containing gas is added via oxygen-containing gas feed line **52**, mixing devices and filtration systems (not shown) may be beneficially employed to aid in the combination of preheated reactor feed **50** and oxygen-containing gas **52**. Oxygen-containing gas feed line **52** may comprise, for example, atmospheric air or 100% oxygen.

The ratio of gases in the feed mixture may be adjusted as necessary to optimize hydrogen cyanide product yield in a reactor **54**, such adjustments are within the ability of one skilled in the art. Product stream **55** comprises hydrogen cyanide, water, and greater than 5% free hydrogen. The presence of significant free hydrogen demonstrates that all of the waste nitrogen oxides are reduced to harmless diatomic nitrogen as a result of this embodiment of the methods of the present invention. Thus, as Figure 2A illustrates, when the methods of the present invention are employed, reactor **54** may act as a second industrial process, eliminating all WOG emissions from gas turbine **41** without the need for any conventional WOG abatement systems.

Figure 2B illustrates an alternative embodiment of a system to produce hydrogen cyanide through the ammoxidation of methane, ammonia, and an oxygen-containing gas over a platinum-based catalyst gauze in a reactor, in accordance with the method of the present invention. It will be evident to one of ordinary skill in the art that, while this embodiment employs the same inventive method as the previous example, the system of Figure 2B utilizes a less complex mechanical configuration to supply oxygen-containing gas to reactor **154**. This mechanical configuration is possible because the reactor (**154**) operates at low pressure, that is less than **10** atmospheres pressure, and therefore does not require high-pressure reactor feeds.

As before, gas turbine **141** bums methane **144** and atmospheric air **145**; however, in this embodiment, there is no external compressor and no shaft work is removed from the gas turbine cycle. The omission of the external compressor provides the benefits of reduced capital costs and simplified operation. In this embodiment, gas turbine **141** acts as a first industrial process, creating WOG exhaust stream **143**. Stream **143** originates from the power turbine section (not shown) of gas turbine **141** at between 1 and 10 atmospheres pressure and from 100°C to 1200°C. Stream **143** is then combined with ammonia from process line **148**, and methane from process line **149** to form preheated reactor feed **150**. Mixing devices, filtration systems, and heat exchangers (not shown) may be beneficially employed to aid in the combination and temperature control of the raw material streams.

Preheated reactor feed **150** is then sent through process line **153** to conventional reactor **154**. As with the previous example, oxygen-containing gas-which may comprise, for example, atmospheric air or 100% oxygen - may be optionally added to process line **153** via oxygen-containing gas feed line **152**. Where oxygen-containing gas is added via oxygen-containing gas feed line **152**, mixing devices, filtration systems, and heat exchangers (not shown) may also be beneficially employed to aid in the combination and temperature control of preheated reactor feed **150** and oxygen-containing gas **152**. The ratio of gases in the feed mixture as well as the overall feed mixture temperature may be adjusted as necessary to optimize hydrogen cyanide product yield in reactor **154**, such adjustments are within the ability of one skilled in the art.

Thus, the embodiment of Figure 2B illustrates how, when a method in accordance with the present invention is employed, reactor **154** may act as a second industrial process, eliminating all WOG emissions from gas turbine **141** without the need for any conventional WOG abatement systems.

It is envisioned that in some situations, it may be beneficial to utilize a fuel mixture comprising at least some non-hydrocarbon compounds, such as ammonia or hydrogen gas, as fuel stream **144** in the embodiment of Figure 2B. In so doing, the concentration of oxides of carbon (COx) in the resulting WOG can be reduced (vs. a pure methane stream, for example), which is desirable in some secondary industrial processes, such as, for example, an HCN reactor that is integrated with a phosphate-based Ammonia recovery process or a conventional Ammonia Oxidation Process (AOP) reactor used in industrial nitric acid production.

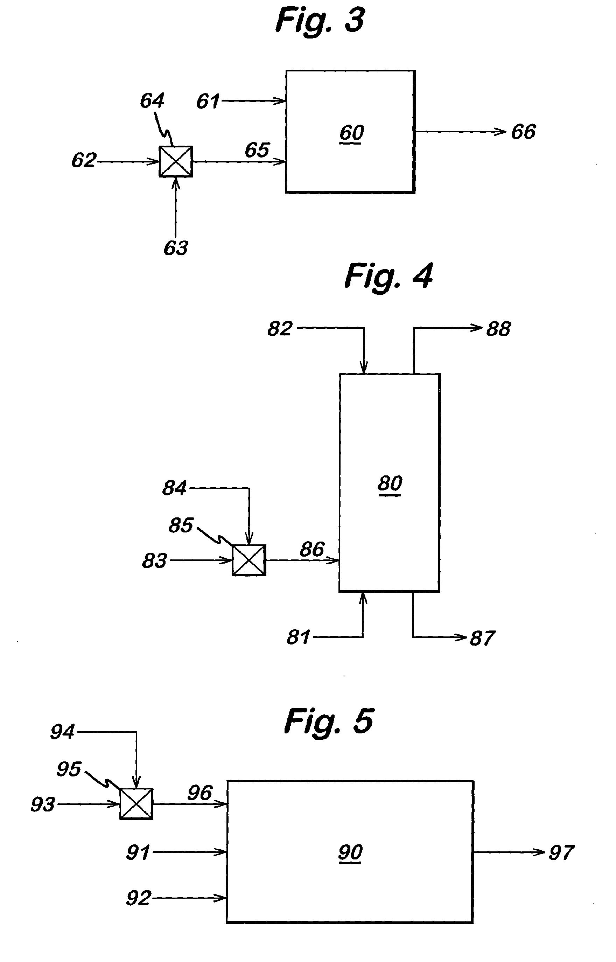

Figure 3 shows a portion of a system for producing phthalic anhydride according to a method in accordance with the present invention. Traditionally, the process for producing phthalic anhydride involves reacting o-xylene and oxygen-containing gas in a catalytic reactor. In this embodiment of the methods of the present invention, the oxygen-containing gas is mixed with a WOG stream produced in a first industrial process before being fed to the catalytic reactor.

In Figure 3, a first industrial process, specifically a thermal oxidizer (not shown) produces a low pressure, low oxygen-content WOG effluent stream **63**. WOG effluent stream **63** and oxygen-containing gas stream **62** are both introduced to mixer **64**. Mixer **64** may be any device suitable for mixing two gaseous process streams, for example, an eductor. Oxygen-containing gas stream **62** is pressurized and may comprise atmospheric air or 100% oxygen. The passage of the pressurized oxygen-containing gas stream **62** through mixer **64** may be used to cause the low-pressure WOG effluent stream **63** to be drawn into mixer **64** by venturi action. WOG stream **63** and the oxygen-containing gas stream **62** are combined in mixer **64** into mixed gas feed stream **65**. Mixed gas feed stream **65** and o-xylene feed stream **61** are then fed to a second industrial process, catalytic reactor **60**. Inside catalytic reactor **60**, mixed gas **65**, comprising WOG and o-xylene, reacts to produce phthalic anhydride. The reaction consumes all waste oxides present in mixed gas feed stream **65**, effectively abating all WOG emissions. Thus, in accordance with this embodiment of a method in accordance with the present invention, catalytic reactor **60** acts as a second industrial process, eliminating all WOG emissions and eliminating the need for traditional WOG abatement equipment.

Shown in Figure 4 is a portion of a system for producing nitric acid according to an embodiment of a method in accordance with the present invention wherein cooled ammonia converter exit gas **81** enters absorber column **80** and is absorbed into cooled process water feed **82** to produce a low concentration aqueous nitric acid product stream **87** and off gas stream **88**. Absorber **80** may be any column known in the art, such as a sieve plate column or bubble cap column, which additionally may typically comprise 20-50 internal trays, and integrated cooling equipment. Conventional extended absorption columns that use a larger number of stages and refrigerated water cooling in their upper sections are preferred for absorber **80** to maximize the yield of nitric acid and to achieve almost complete NOₓ capture efficiency. Bleaching air **83** is added to absorber **80** via process feed line **86** to drive residual NOₓ to nitric acid. In an alternate embodiment (not shown), dilute nitric acid may be recirculated through absorber **80** to improve operation of the absorber.

As shown in the embodiment of Figure 4, WOG stream **84** is combined with bleaching air **83** prior to its introduction to absorber **80**. In this embodiment, WOG stream **84** typically comprises an absorber off gas stream containing WOG from a first industrial process wherein cyclohexane is used to produce adipic acid. WOG stream **84** usually is comprised of 500-1500 ppm NOₓ. WOG stream **84** and bleaching air **83** are combined in mixer **85** before they are introduced to absorber **80** via process feed line **86**. Mixer **85** may be any device known in the art to mix industrial gas streams, for example, an eductor or static mixer. In a further alternate embodiment (not shown), it may be desirable to reduce the N₂O content of WOG stream **84** before it is introduced to mixer **85**. Any process known in the art for reducing N₂O content may be employed; for example, utilization of silver-based catalyst at 500-550°C is known to convert N₂O to a mixture of NO₂, NO, N₂ and O₂. The absorber acts as a second industrial process wherein the nitrogen oxides present in WOG stream **84** are converted into nitric acid and exit absorber 80 via product stream **87**.

Figure 5 illustrates another embodiment of a process in accordance with the present invention, shown in the context of a furnace-based system for producing sulfuric acid. Traditionally, oxygen-containing gas, hydrocarbon fuel and a sulfur bearing residue stream are fed to a furnace to produce sulfur oxides for use in downstream processing to create products such as SO₃ or H₂SO₄. According to this embodiment of a process in accordance with the present invention, a WOG stream comprising sulfur oxides is combined with the oxygen-containing gas prior to introduction to the furnace.

Shown in the embodiment of Figure 5, sulfur-bearing residue stream **92**, hydrocarbon fuel **91** for example methane, and blended stream **96** are fed to furnace **90**. Mixer **95** is used to combine WOG stream **94** comprising sulfur oxides and oxygen-containing gas stream **93** to produce blended stream **96**. Mixer **95** may be any device suitable for mixing two gaseous process streams, for example, an eductor. In the process shown in Figure 5, WOG stream **94** comprising sulfur oxides is an effluent stream from a steel plant burning high sulfur content coal fuel. Sulfur-bearing residue stream **92**, hydrocarbon fuel **91**, and blended stream **96** are introduced to furnace **90**, and burned to create sulfur oxides that exit furnace **90** in product line **97**. The sulfur oxides present in WOG stream **94** pass through furnace **90** unchanged and exit via product line **97** as additional product.

The yield improvement seen in furnace **90** due to the addition of preformed sulfur oxides is not the only benefit imparted by the method of the present invention on the sulfuric acid process shown in Figure 5. Because WOG stream **94** often exhibits an elevated temperature, mixed stream **96** will be effectively preheated before its introduction to furnace **90**, and will result in a lower hydrocarbon fuel requirement in order to maintain the high temperatures needed in furnace **90**. Moreover, while the WOG stream containing sulfur oxides may be added to this process downstream of the furnace in oxide-containing product line **97**, its introduction to the oxygen-containing gas feed via mixer **95** allows any residual oxygen present in WOG stream **94** to be utilized in furnace **90**, thereby requiring less oxygen-containing gas from process line **93** to run the furnace. In some embodiments, it may also be advantageous to mix a portion of the hot WOG stream **94** with residue stream **92** to improve atomization of the residue feed.

The present invention, therefore, is well adapted to carry out the objects and attain both the ends and the advantages mentioned, as well as other benefits inherent therein. While the present invention has been depicted, described, and is defined by reference to particular embodiments of the invention, such references do not imply a limitation on the invention, and no such limitation is to be inferred. The depicted and described embodiments of the invention are exemplary only and are not exhaustive of the scope of the invention. Consequently, the invention is intended to be limited only by the spirit and scope of the appended claims.

## Claims

1. A method for abating waste oxide gases from a waste oxide gas stream, the method comprising:
(a) providing a first industrial process, the first industrial process producing a waste oxide gas stream, the waste oxide gas stream comprising at least one waste oxide gas selected from the group consisting of nitrogen oxides, sulfur oxides and carbon oxides;
(b) providing a second industrial process, the second industrial process being a different process than the first industrial process, the second industrial process abating the quantity of said waste oxide gas stream, from the first industrial process, when said waste oxide gas stream is fed to said second industrial process as a feed stream; and
(c) feeding at least a portion of said waste oxide gas stream, from the first industrial process, as a feed stream, to said second industrial process.

2. The method for abating waste oxide gases from a waste oxide gas stream according to claim 1 wherein the first industrial process is chosen from the group consisting of a chemical manufacturing process, a combustion process, a process comprising a gas turbine, a high-temperature industrial manufacturing process, a process comprising an air compressor, a co-generation process, and a traditional waste oxide abatement system.

3. The method for abating waste oxide gases from a waste oxide gas stream according to claim 1 wherein the second industrial process is chosen from the group consisting of a hydrogen cyanide production process;
a bleaching process;
a carbon bed desorption process;
an oxidation process;
an oxidative dehydrogenation of hydrocarbons process;
an oxygen addition process;
an ammoxidation process;
an air stripping process;
a partial oxidation of hydrocarbon process;
a sulfuric acid regeneration process;
a reaction of tert-butanol, isobutene, iso-butane, iso-butyraldehyde or the methyl ether of tert-butanol to yield (meth)acrolein and/or (meth)acrylic acid;
a phthalic anhydride reaction;
a reaction of butadiene; and
a reaction of indanes.

4. The method for abating waste oxide gases from a waste oxide gas stream according to claim 1 wherein the second industrial process is a process wherein at least one of hydrogen, carbon oxides, nitrogen oxides, ammonia, hydrocarbons and oxygen is routinely present.
